(19) Europäisches Patentamt
European Patent Office
Office européen des brevets

(11) **EP 3 991 729 B1**

(12) **EUROPEAN PATENT SPECIFICATION**

(45) Date of publication and mention of the grant of the patent:
**25.03.2026 Bulletin 2026/13**

(21) Application number: **20831767.7**

(22) Date of filing: **28.06.2020**

(51) International Patent Classification (IPC):
***A61K 31/436*** (2006.01) ***A61P 31/12*** (2006.01)
***A61P 1/16*** (2006.01) ***A61K 31/513*** (2006.01)
***A61K 31/522*** (2006.01) ***A61K 31/675*** (2006.01)
***A61K 45/06*** (2006.01) ***A61P 31/20*** (2006.01)
***A61P 35/00*** (2006.01)

(52) Cooperative Patent Classification (CPC):
(C-Sets available)
**A61P 1/16; A61K 31/436; A61K 31/513; A61K 31/522; A61K 31/675; A61K 45/06; A61P 31/12; A61P 31/20; A61P 35/00;** Y02A 50/30
(Cont.)

(86) International application number:
**PCT/CN2020/098635**

(87) International publication number:
**WO 2020/259706 (30.12.2020 Gazette 2020/53)**

(54) **USE OF AMLEXANOX IN PREPARING ANTI-HEPATITIS VIRUS DRUG**

VERWENDUNG VON AMLEXANOX ZUR HERSTELLUNG EINES ARZNEIMITTELS GEGEN DAS HEPATITIS-VIRUS

UTILISATION DE L'AMLEXANOX DANS LA PRÉPARATION D'UN MÉDICAMENT CONTRE LE VIRUS DE L'HÉPATITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2019 CN 201910556045**

(43) Date of publication of application:
**04.05.2022 Bulletin 2022/18**

(73) Proprietor: **Marine Biomedical Research Institute Of Qingdao Co., Ltd.**
**Qingdao, Shandong 266071 (CN)**

(72) Inventors:
- **WANG, Xin**
  **Qingdao, Shandong 266061 (CN)**
- **YANG, Jinbo**
  **Qingdao, Shandong 266061 (CN)**
- **XU, Ximing**
  **Qingdao, Shandong 266061 (CN)**
- **LI, Li**
  **Qingdao, Shandong 266061 (CN)**
- **ZHAO, Chenyang**
  **Qingdao, Shandong 266061 (CN)**
- **WANG, Yuxin**
  **Qingdao, Shandong 266061 (CN)**
- **WU, Juan**
  **Qingdao, Shandong 266061 (CN)**
- **ZHAO, Lili**
  **Qingdao, Shandong 266061 (CN)**
- **YANG, Menglin**
  **Qingdao, Shandong 266061 (CN)**

(74) Representative: **Tiburzi, Andrea et al**
**Barzanò & Zanardo Roma S.p.A.**
**Via Piemonte 26**
**00187 Roma (IT)**

(56) References cited:
**WO-A1-2016/201243** **WO-A2-2006/133099**
**JP-A- 2010 083 871** **US-A1- 2016 289 684**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/436, A61K 2300/00;**
**A61K 31/496, A61K 2300/00;**
**A61K 31/505, A61K 2300/00;**
**A61K 31/513, A61K 2300/00;**
**A61K 31/52, A61K 2300/00;**
**A61K 31/522, A61K 2300/00;**
**A61K 31/536, A61K 2300/00;**
**A61K 31/551, A61K 2300/00;**
**A61K 31/675, A61K 2300/00;**
**A61K 31/7068, A61K 2300/00;**
**A61K 31/7072, A61K 2300/00;**
**A61K 31/708, A61K 2300/00**

## Description

### Technical Area

The invention relates to the pharmaceutical field, in particular to the use of amlexanox for preparing anti-hepatitis virus drugs.

### Background Technology

Hepatitis virus infection often causes viral hepatitis, and related pathogens mainly include hepatitis A virus (HAV), hepatitis B virus (HBV), hepatitis C virus (HCV), hepatitis D virus (HDV), hepatitis E virus (HEV), and hepatitis G virus (HGV), among which the first four kinds have a relatively wide infection range and cause extensive damage. Therapeutic drugs for hepatitis virus infection are diverse. The main mechanism is to inhibit virus-encoded DNA polymerase during reverse transcription of viral replication, disrupt the stability of virus-encoded RNA during virion assembly, or indirectly inhibit viral replication by acting on the host's interferon receptor.

Taking the virus hepatitis B as an example, the most common drugs used in clinical practice are DNA polymerase inhibitors. At present, DNA polymerase inhibitors on the market are mainly nucleosides or nucleotide analogs, such as lamivudine, entecavir, adefovir, and tenofovir. These nucleosides or nucleotide analogs inhibit viral DNA synthesis by inhibiting viral polymerase from achieving an antiviral effect. Long-term administration of lamivudine, adefovir, and entecavir has the potential toxicity to cause hepatitis B virus mutation. Moreover, DNA polymerase inhibitors do not directly inhibit the expression of hepatitis B surface antigen (HBsAg).

High-level HBsAg concentration in bodies can induce T cell depletion in patients infected by chronic hepatitis B virus, and HBsAg antibodies are not produced by injecting HBsAg-based vaccines. Based on these drugs' defects and the characteristics of hepatitis B infection, HBsAg antibodies levels may be improved by giving vaccines in clinical treatments when the testing result of HBsAg in serum is negative. Therefore, it could effectively treat chronic hepatitis B virus infection by reducing HBsAg levels. Some drugs in clinical trial periods have an unclear treatment effect. These drugs' targets are viral HBcAg and viral RNA, or they act by neutralizing viral surface antigen (HBsAg) by specific antibodies. For example, Arrowhead Pharmaceuticals uses RNA interference (RNAi) technology represented by the drug under development, JNJ-3989, which can reduce 99% HBsAg at most. Still, RNAi technology requires a better drug delivery system and strict storage technology, and also, these kinds of drugs cannot be taken orally and come at a greater cost.

Interferon is also commonly used in the treatment of hepatitis B. It implements indirect anti-hepatitis B infection effects through the immune system and targets interferon receptors, including short-acting interferon (normal interferon) and long-acting interferon (pegylated interferon). For example, pegylated interferon is used to treat hepatitis B and hepatitis C. Interferon has noticeable side effects, can cause high fever, etc., and usually requires injection administration. Patient compliance is poor, and it cannot directly and effectively reduce HBsAg levels in patients with high levels. In addition, interferon therapy is usually not recommended for hepatitis B infected patients with liver fibrosis

US2016/289684 relates to the use of a IKKε inhibitor such as amlexanox for treating a viral infection.

WO2006/133099 discloses a method for treating or preventing a virus-mediated disease, which comprises administering to cells a pharmaceutical composition that comprises an effective amount of a microbicide and a carrier.

WO2016/201243 discloses the use of nucleosides or nucleotide such as lamivudine, entecavir, tenofovir for the treatment of hepatitis B.

### Description of the Invention

In the first aspect of the invention, the novel use of amlexanox for preparing antiviral drugs is provided.

In some protocols, antiviral drugs are drugs to prevent and/or treat hepatitis virus infection or drugs to prevent and/or treat diseases caused by hepatitis virus infection.

The virus is a DNA virus and/or RNA virus in some preferred protocols, preferably a hepatitis B virus.

Diseases caused by hepatitis virus infection are chosen from acute hepatitis, chronic hepatitis, cirrhosis, hepatic fibrosis, hepatic carcinoma, hepatic injury, and/or other medically defined diseases.

In some protocols, the form of amlexanox includes pharmaceutically acceptable salt, hydrate, solvate, or prodrug, providing that the pharmaceutically acceptable salt, hydrate, solvate, or prodrug has a similar anti-hepatitis viral effect.

In the second aspect of the invention, a pharmaceutical composition is provided, comprising amlexanox, additional active antiviral substances and pharmaceutically acceptable carriers, wherein the active antiviral substances are one or more nucleosides or nucleotide analogs chosen from lamivudine, zidovudine, stanvudine, tenofovir, entecavir, abacavir, zalcitabine, emtricitabine, dideoxyinosine, nevirapine, delavirdine, efavirenz, etravirine, rilpivirine, and preferably one or more of lamivudine, tenofovir, or entecavir.

According to one aspect which is not part of the invention, the active antiviral substances are transcription or reverse transcription inhibitors, DNA polymerase inhibitors, reverse transcriptase inhibitors, capsid assembly modulators, capsid protein inhibitors, viral mRNA inhibitors, or combinations thereof.

In the third aspect of the invention, the use of pharmaceutical compositions for preparing drugs for preventing and/or treating viral infections or for preventing and/or treating diseases caused by viral infections is provided.

Ideally, the virus should be a DNA virus and/or RNA virus, preferably hepatitis B virus.

The conditions resulting from hepatitis virus infection are acute hepatitis, chronic hepatitis, cirrhosis, hepatic fibrosis, liver cancer, liver injury, and/or other medically defined conditions.

In other preferred protocols, the antiviral drug is an oral preparation or injectable preparation; the oral preparation is preferably a tablet, capsule, granule, and/or oral solution.

In the fourth aspect of the present invention, the pharmaceutical composition as defined above for use in the prevention and/or treatment of viral infections or in the prevention and/or treatment of diseases caused by hepatitis virus infection is provided.

In a further aspect which is not part of the present invention, a method is provided for preventing and/or treating viral infections or for preventing and/or treating diseases caused by viral infections, in particular applying an amlexanox carrier or the pharmaceutical composition to a subject (as described in the second aspect).

Ideally, the virus should be a DNA virus or RNA virus, preferably hepatitis B virus.

Preferably, the subject is human.

The diseases caused by hepatitis virus infection are acute hepatitis, chronic hepatitis, liver cirrhosis, liver cancer, liver injury, and/or other medically defined diseases.

Preferably, the method is applied orally.

Preferably, the application dose of the method is 1~500 mg/day, preferably 50~500 mg/day; optimally, 50~200 mg/day.

Preferably, the method includes once or more daily applications (e.g., 1, 2, 3, or 4 applications daily) or multiple daily applications (e.g., every 1, 2, or 3 days).

Preferably, the method is applied at least once daily (e.g., 1, 2, 3, or 4 times daily), and the total daily dose is 1~500 mg/day, preferably 50~500 mg/day; optimally, 50~200 mg/day.

In the fifth aspect of the invention, a method for non-therapeutic inhibition of viral infection in vitro is provided, which includes: culturing cells that have been transfected by viral genome DNA (e.g., cells that have been transfected by viral genome DNA for 16~24 hours) in the presence of amlexanox, thereby inhibiting viral infection.

Preferably, the virus is defined as before.

Preferably, incubate for 24 to 144 hours.

It is understood that, within the scope of the invention, each of the aforementioned technical features and each of the technical features specifically described below (e.g., embodiments) may be combined to form a new or preferred technical solution. Due to limitations, it has not been repeated here. The references to methods of treatment in the detailed description of the invention in this description are to be interpreted as references to the compounds, pharmaceutical compositions and medicaments of the present invention for use in a method for treatment of the human (or animal) body by therapy.

Attached Figure Description:

Figure 1 shows that amlexanox inhibits hepatitis B virus genomic DNA replication in a cellular model.

Figure 2 shows that amlexanox reduces HBsAg levels in a cellular model.

Figure 3 shows that amlexanox reduces HBsAg levels in a mouse venous hypertension model.

Figure 4 shows that amlexanox reduces hepatitis B virus core antigen levels in a mouse venous hypertension model.

Figure 5 shows that amlexanox reduces hepatitis B virus DNA replication in a mouse venous hypertension model.

Figure 6 shows the combination of amlexanox with clinically used nucleotide analog drugs to reduce HBsAg levels in cellular models.

Figure 7 shows the dose-response relationship of hepatitis B virus inhibition by amlexanox in a mouse venous hypertension model.

Figure 8 shows amlexanox's efficacy and dose-response relationship in inhibiting subtype A hepatitis B virus in a mouse venous hypertension model.

Figure 9 shows the effect of amlexanox on innate immunity in mice.

Figures 10, 11, and 12 show that amlexanox inhibits hepatitis B virus replication in the HepAD38 cell model.

Figure 13 shows that amlexanox reduces HBsAg levels in a cellular model.

Figure 14 shows that amlexanox reduced hepatitis B virus core antigen levels in a cellular model (HepAD38 cells).

Figure 15 shows the dose-response relationship of hepatitis B virus inhibition by amlexanox in a mouse venous hypertension model.

Figure 16 shows that amlexanox reduced the degree of liver injury in a mouse venous hypertension model.

Figure 17 shows the inhibitory effect of amlexanox on 3TC/ETV-resistant HBV.

Figure 18 shows that amlexanox reduced hepatitis B surface antigen in a transgenic mouse model.

Figure 19 shows that amlexanox does not inhibit the number of immune cells in mice.

**Specific Implementation Mode**

After extensive and in-depth research, the inventor accidentally discovered a novel application of the drug amlexanox for the preparation of antiviral drugs. On this basis, the inventor completes the invention.

**Term Description**

The methods and techniques of the invention are generally based on the traditional methods known in the field unless otherwise stated. The nomenclature and experimental methods and techniques related to biology, pharmacology, medicine, and medicinal chemistry described are known and commonly used in this field. Standard techniques are used for chemical synthesis methods, chemical analysis methods, pharmaceutical preparation methods, preparation methods and delivery methods, and detection or testing methods.

Unless otherwise stated, the scientific and technical terms used herein shall have the meanings generally understood by ordinary technical personnel in the field.

**Amlexanox and Antiviral Effect**

Amlexanox (AMLE) is clinically used to treat oral ulcers, bronchial asthma, etc. There is no report on the drug against liver virus infection. Amlexanox was originally developed by Takeda Pharmaceutical Company (Japan), and its action mechanism was unclear. It was initially developed as an oral tablet for the treatment of bronchial asthma and was included in the Japanese Pharmacopoeia, after which it was developed for anti-oral ulcer function and widely used in clinical practice in Europe and the United States. In China, amlexanox is approved for the treatment of recurrent oral ulcers in the form of a paste. In 2013, amlexanox was found to have an inhibitory effect on TBK1/IKKε, and its clinical trials for the treatment of gastric ulcer, obesity, and nonalcoholic fatty liver disease are ongoing. The anti-hepatic viral effect of amlexanox has not been reported.

The structural formula for amlexanox is shown in Equation I:

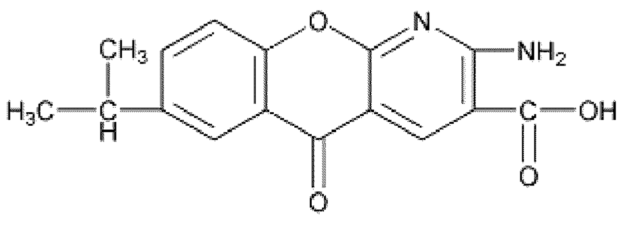

(I)

As used herein, the term "compound of the invention" refers to the compound shown in Equation I. The term also includes pharmaceutically acceptable salts of transitive I compounds.

In the invention, the term "amlexanox" includes the formula I compound and pharmaceutically acceptable salt.

As used herein, the term "pharmaceutically acceptable salt" refers to a salt formed from the reaction of the above Equation I compound with inorganic acids, organic acids, alkali metals, or alkaline earth metals, etc. These salts include, but are not limited to: (1) salts formed with inorganic acids such as hydrochloric acid, hydrobromic acid, hydroiodic acid, sulfuric acid, nitric acid, and phosphoric acid; (2) salts formed with organic acids such as acetic acid, lactic acid, citric acid, succinic acid, fumaric acid, gluconic acid, benzoic acid, methane sulfonic acid, ethane sulfonic acid, benzenesulfonic acid, P-toluene sulfonic acid, oxalic acid, succinic acid, tartaric acid, maleic acid, or arginine; (3) other salts, including salts formed with alkali metals or alkaline earth metals (such as sodium, potassium, calcium, or magnesium), ammonium salts or water-soluble amines (such as N-methyl glucosamine), low-grade alkanol ammonium salts, and other pharmaceutically acceptable amines (such as methylamine, ethylamine, propionate, dimethylamine, trimethylamine, diethylamine, triethylamine, tert-butylamine salt, ethylenediamine salt, hydroxyethyl-amine salt, dihydroxy-ethyl-amine salt, trihydroxy-ethyl-amine salt, and amine salts formed by morpholine, piperazine and lysine respectively).

The term "prodrug" refers to the compound contained in the general formula of the invention and the salt or solution composed of the compound, which is metabolized or chemically reacted in the patient when taken by an appropriate method. The compounds include but are not limited to the carboxylic acid ester, carbonate, phosphate ester, nitrate ester, sulfate ester, sulfone ester, sulfoxide ester, amino compound, carbamate, azo compound, phosphoramide, glucoside, ether, acetal, and other forms.

The term "therapeutically effective dose" refers to any amount of a drug as described below that, when used alone or in combination with another therapeutic agent, promotes disease resolution, which is manifested by a reduction in the severity of disease symptoms, an increase in the frequency and duration of symptom-free periods, or prevention of disorders or incapacitations caused by the disease. The "therapeutically effective dose" of a drug of the invention also includes a "preventive effective dose." A "preventive effective dose" is any of the following amounts of a drug that, when administered alone or in combination with another therapeutic agent, inhibits the occurrence or recurrence of a disease in a subject at risk of developing or suffering from the disease recurrence.

As used herein, when referring to a specific enumerated value, the term "approximately" means that the value may vary by no more than 1% from the enumerated value. For example, as used herein, the expression "about 100" includes all values between 99 and 101 (e.g., 99.1, 99.2, 99.3, 99.4, etc.).

As used herein, the terms "include" or "comprise" may be open, semi-closed, or closed. In other words, the term also includes "basically comprise ..." or "consist of ...".

In the invention, it is evident for the technicians in the field that the effective in vivo dose and the specific mode of administration will vary according to the mammalian species, weight and age treated, the specific compounds used, and the specific purpose for which these compounds are used. The effective dose level (that is, the dose level necessary to achieve the desired effect) can be determined by the technicians in the field based on conventional pharmacological methods. Generally, human clinical application of the product starts with a lower dose level, followed by a continuous increase of the dose level until the desired effect is achieved. Alternatively, acceptable in vitro studies may be employed with existing pharmacological methods to establish useful doses and routes of administration for compositions identified by the method.

## **Drug** Composition **and Administration Method**

Because the compounds of the invention, such as amlexanox, have excellent antiviral ability, the compounds of the invention and various crystal forms, pharmaceutically acceptable inorganic or organic salts, hydrates or solvent complexes, and pharmaceutical combinations containing the compound of the invention as the main active ingredient can be used for the treatment, prevention, and alleviation of hepatitis virus infection and/or diseases caused by hepatitis virus infection. According to the present technology, the compound can be used for treating the following diseases: acute hepatitis, chronic hepatitis, liver cirrhosis, liver cancer, liver injury, or other medically defined diseases.

The pharmaceutical compositions of the invention include the compound of the invention or its pharmacologically acceptable salt and the pharmacologically acceptable excipient or carrier within the safe and effective dose range. "Safe and effective dose" refers to the amount of compound that is sufficient to significantly improve the condition without causing serious side effects. Generally, a pharmaceutical composition contains 1 to 2000mg of the compound/agent of the invention, preferably 10 to 500 mg (preferably 25 to 200mg such as 25 to 100 or 50 to 200 mg) of the compound/agent of the invention. Preferably, the "one dose" is a capsule or tablet. The invention’s compound may also include other active antiviral substances (such as those previously defined).

"Pharmaceutically acceptable carrier" means one or more compatible solid or liquid fillers or gel substances, which are suitable for human use and must be of sufficient purity and low enough toxicity. "Compatibility" herein refers to the ability of components in the combination to interact with and between the compounds of the invention without

significantly reducing the efficacy of the compounds. Some examples of pharmaceutically acceptable carriers include cellulose and its derivatives (such as sodium carboxymethylcellulose, sodium ethylcellulose, cellulose acetate, etc.), gelatin, talc, solid lubricants (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween®), wetting agent (such as sodium lauryl sulfate), colorant, flavoring agent, stabilizer, antioxidant, preservative, pyrogen-free water, etc.

The compound or drug composition of the invention is applied in a manner not specifically limited, and representative application methods include (but are not limited to): oral, intratumoral, rectal, parenteral (intravenous, intramuscular, or subcutaneous), and local administration. 1) Solid dosage forms for oral administration include capsules, tablets, pills, powder, and granules. In these solid dosage forms, the active compound is mixed with at least one conventional inert excipient (or carrier), such as sodium citrate or dibasic calcium phosphate, or with: (a) fillers or compatibilizers, such as starch, lactose, sucrose, glucose, mannitol, and silicic acid; (b) adhesives, such as hydroxy methylcellulose, alginate, gelatin, polyvinylpyrrolidone, sucrose, and acacia; (c) moisturizers, such as glycerol; (d) disintegrants, such as agar, calcium carbonate, potato starch or cassava starch, algal acid, certain complex silicates, and sodium carbonate; (e) slow solvents, such as paraffin; (f) absorption accelerators, such as quaternary compounds; (g) humectants, such as wax alcohol and monostearate; (h) adsorbents, such as kaolin; and (i) lubricants, such as Talc, calcium stearate, magnesium stearate, solid polyethylene glycol, sodium lauryl sulfate, or a mixture thereof. In capsules, tablets, and pills, the dosage form may also contain a buffer. Solid dosage forms such as tablets, sugar pills, capsules, pills, and granules may be prepared from coatings and shells, such as bowel coatings and other materials known in this field. They may contain opaque agents, and the release of active compounds or compounds from such combinations may be delayed in a certain part of the digestive tract. Examples of available entrapment components are polymeric substances and waxy substances. The active compound may also form microcapsules with one or more of the above excipients when necessary. 2) Liquid dosage forms for oral administration include pharmaceutically acceptable lotions, solutions, suspensions, syrups, or tinctures. In addition to active compounds, liquid dosage forms may include inert diluents routinely used in the field, such as water or other solvents, solubilizers, and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, and sesame oil or mixtures thereof. In addition to these inert diluents, compositions may also contain aids such as wetting agents, emulsifiers and suspending agents, sweeteners, flavoring agents, and spices. In addition to the active compounds, the suspension may contain suspending agents such as ethoxylated isooctadecanol, polyoxyethylene sorbitol, and sorbitan, microcrystalline cellulose, methanolic aluminum, and agar, or a mixture of these, etc. 3) Combinations for parenteral injection may contain physiologically acceptable sterile aqueous or anhydrous solutions, dispersions, suspensions, or emulsions, and sterile powders for redissolution into sterile injectable solutions or dispersions. Suitable aqueous and non-aqueous carriers, diluents, solvents, or excipients include water, ethanol, polyols, and their appropriate mixtures. The dosage forms of the invention compounds for topical administration include ointments, powders, patches, sprays, and inhalants. The active ingredient is aseptically mixed with a physiologically acceptable carrier and any preservative, buffer, or propellant, as required.

The invention's compound can be administered alone or in combination with other pharmaceutically acceptable compounds.

When using a drug combination, a safe and effective amount of the compound of the invention is applied to mammals (such as humans) requiring treatment, in which the dose at the time of application is the effective dose considered pharmaceutically, and for a person of 60kg body weight, the daily dose is usually 1 to 2000mg, preferably 20 to 500mg (ideally 50 to 200mg). Of course, the specific dose shall consider the route of administration, patient health status, and other factors within the range of skilled physician skills.

**The invention has the following main beneficial effects:**

(a) Amlexanox has a significant antiviral effect;

(b) Amlexanox has proven to be safe;

(c) Amlexanox can be used either alone or in combination with other related antiviral drugs, with better results than existing anti-hepatitis virus drugs and good commercial prospects.

Combined with the specific implementation, the invention is further described in the following. It is understood that these embodiments are intended only to describe the invention and not to limit the scope of the invention. The following test methods without specific conditions are generally implemented according to the conventional conditions or the

conditions recommended by the manufacturer. Unless otherwise stated, percentages and copies are calculated by weight.

**Implementing Example 1** Amlexanox inhibits hepatitis B virus genomic DNA replication in a cellular model.

Hepatocyte HepG2 cells with good growth status were seeded in 6-well plates at $10^6$ cells/well, and after overnight, 2μg of linear HBV viral genomic DNA (genotype B) was transfected into each well using lipofectamine 2000 to establish a viral infection cell model. Amlexanox, lamivudine, entecavir, or tenofovir were added at a final concentration of 10μM for 16-24h after transfection. An equal volume of DMSO (1/1000, v/v) was added to the control group. After 96 hours, cells were divided into two groups, with genomic DNA extracted from one. From the other, free DNA was extracted using HIRT extraction, and linear DNA was removed using DNA exonuclease (NEB). Circular HBV DNA and genomic GAPDH were detected by quantitative PCR. The highest value of circular HBV DNA was defined as 100% (n=5).

From the comparison in Figure 1, it can be seen that amlexanox inhibits hepatitis B virus genomic DNA replication in cell models with effects comparable to existing clinical drugs.

**Implementing Example 2** Amlexanox reduces hepatitis B virus surface antigen levels in a cell model and is more effective than existing clinical drugs.

Hepatocyte HepG2 cells with good growth status were seeded in 6-well plates at $10^6$ cells/well and, after being left overnight, 2μg of linear HBV viral genomic DNA (genotype B) was transfected into each well using lipofectamine 2000 to establish a viral infection cell model. Amlexanox, lamivudine, entecavir, or tenofovir were added at a final concentration of 10μM 16-24h after transfection. An equal volume of DMSO (1/1000, v/v) was added to the control group. Forty-eight hours after transfection, cells from each well were passaged into one 6cm culture plate. After transfection, HBsAg levels in the culture supernatant were quantified by ELISA 96h.

From the comparison in Figure 2, it can be seen that amlexanox reduces HBsAg levels in cell models, and its effect is superior to that of existing clinical drugs.

**Implementing Example 3** Amlexanox reduces HBsAg levels in a mouse venous hypertension model.

Wild-type mice (C57/B6) were rapidly injected with 10μg/mouse of hepatitis B virus genomic DNA (genotype B) by tail vein injection in a short period. Viral infection was established using a venous hypertension model randomly divided into groups of 5 mice each. From the second day, oral gavage administration of amlexanox (AMLE, 50 mg/kg/day), or lamivudine (530 mg/kg/day), or an equal volume of an equal concentration of cosolvent cyclodextrin (control). On the 9th day after infection, about 100μl/mouse of blood was harvested from the tail to separate serum, and the concentration of HBsAg in peripheral blood was detected by ELISA. The OD value at 450nm was measured on a microplate reader.

From the comparison results in Figure 3, it can be seen that amlexanox reduced HBsAg levels in a mouse model of venous hypertension, and its effect is better than that of lamivudine.

Implementing Example 4 Amlexanox reduced hepatitis B virus core antigen levels in a mouse venous hypertension model, and its effect was comparable to that of lamivudine.

Wild-type mice (C57/B6) were rapidly injected with 10μg/mouse of hepatitis B virus genomic DNA (genotype B) by tail vein injection in a short period. Viral infection was established using a venous hypertension model randomly divided into groups of 5 mice each. From the second day, oral gavage administration of amlexanox (AMLE, 50 mg/kg/day), or lamivudine (530 mg/kg/day), or an equal volume of an equal concentration of cosolvent cyclodextrin (control). On the 9th day after infection, about 100μl/mouse of blood was harvested from the tail to separate serum. The concentration of hepatitis B virus core antigen (HBeAg) in peripheral blood was measured by ELISA. The OD value at 450nm was measured on a microplate reader.

From the comparison results in Figure 4, it can be seen that amlexanox reduced hepatitis B virus core antigen levels in a mouse model of venous hypertension, and its effect was comparable to that of lamivudine.

Implementing Example 5 Amlexanox reduces hepatitis B virus DNA replication in a mouse venous hypertension model.

Wild-type mice (C57/B6) were rapidly injected with 10μg/mouse of hepatitis B virus genomic DNA (genotype B) by tail vein injection in a short period. Viral infection was established using a venous hypertension model randomly divided into groups of 5 mice each. From the next day, oral gavage administration of amlexanox (AMLE, 50 mg/kg/day), lamivudine (530 mg/kg/day), entecavir (0.03 mg/kg/day) or tenofovir (530 mg/kg/day), or an equal volume of the same concentration of cosolvent cyclodextrin (control) was performed. On the 11th day after infection, the mice were sacrificed, the livers of the mice were isolated, DpnI was transferred to exclude source DNA, and HBV genomic DNA in the livers of the mice was detected by fluorescence quantitative PCR (n=5).

From Figure 5, it can be seen that in animal models, AMLE can significantly inhibit the load of viral DNA, and its effect is not lower than that of clinically used nucleotide-like drugs lamivudine and entecavir.

**Implementing Example 6,** Amlexanox is used in combination with a clinically used nucleotide analog to reduce hepatitis B virus surface antigen levels in a cell model.

Well-grown hepatocyte HepG2 cells $10^6$ cells/well were seeded in 6-well plates, and after overnight, 2μg of linear HBV viral genomic DNA (genotype B) was transfected per well using Lipofectamine 2000. 16 to 24 hours after transfection, compounds were added at a final concentration of 10μM as shown ("+" represents the presence of a drug or active

substance). An equal volume of DMSO (1/1000, v/v) was added to the control group. Forty-eight hours after transfection, cells from each well were passaged into one 6cm culture plate. HBV surface antigen (HBsAg) in the culture medium was detected by ELISA 96h after transfection.

As shown in Figure 6, in the cell model, the combination of amlexanox with lamivudine or tenofovir is more effective than amlexanox alone and significantly better than lamivudine or tenofovir alone.

**Implementing Example 7** Dose-response relationship of amlexanox inhibition of hepatitis B virus in a mouse model of venous hypertension.

Wild-type mice (C57/B6) were rapidly injected with 10μg/mouse of hepatitis B virus genomic DNA (genotype B) by tail vein injection in a short period. Viral infection was established using a venous hypertension model randomly divided into groups of 5 mice each. From the second day, oral gavage administration of ammonia was performed, daily dose as shown in Figure 7, or an equal volume of an equal concentration of cosolvent cyclodextrin (control). On the 9th day after infection, about 100μl of blood was harvested from each animal, serum was separated, and the concentration of HBsAg in peripheral blood was measured by ELISA.

It can be seen from the comparison in Figure 7 that with the increase of ammonia concentration, its ability to inhibit the hepatitis B virus in the venous hypertension mouse model is enhanced. The dose-effect relationship is most apparent below the dose of 10mg/kg, and the significant effect is at the dose of 10-50 mg/kg.

**Implementing Example 8** Effect and dose-response relationship of amlexanox in inhibiting subtype A hepatitis B virus in a mouse model of venous hypertension.

Wild-type mice (C57/B6) were rapidly injected with 10μg/mouse of viral genomic DNA (genotype A) by tail vein injection in a short period, and viral infection was established using a venous hypertension model and randomly divided into groups. From the second day after injection, according to the compound concentration-time curve, lamivudine (3TC, 530 mg/kg/day, once a day), tenofovir (TD, 530 mg/kg/day, once a day), or the designated concentration of amlexanox (4, 8, and 16 mg/kg/time, twice a day) or an equal volume of an equal concentration of cosolvent cyclodextrin (NC) were administered orally by gavage. On the 9th day after infection, about 100μl of blood was harvested from each animal, serum was separated, and the concentration of HBeAg in peripheral blood was measured by ELISA.

As can be seen from the comparison in Figure 8, with the increase in the concentration of amlexanox, its ability to inhibit hepatitis B virus is enhanced in a mouse model of venous hypertension, and the dose-response relationship is most pronounced between 4 and 8 mg/kg, and 16 mg/kg is still effective.

**Implementing Example** 9 Effect of amlexanox on innate immunity in mice.

Wild-type mice (C57/B6) were rapidly injected with 10μg/mouse of viral genomic DNA (genotype A) by tail vein injection in a short period, and viral infection was established using a venous hypertension model and randomly divided into groups. From the second day, according to the compound concentration-time curve, lamivudine (3TC, 530 mg/kg/day, once a day), tenofovir (TD, 530 mg/kg/day, once a day), or the indicated concentrations of amlexanox (4, 8, and 16 mg/kg/time, twice a day) or an equal volume of an equal concentration of cosolvent cyclodextrin (NC) were administered orally by gavage. On the 9th day after infection, about 100μl/mouse of blood was harvested from the tail, serum was separated, and the concentration of type I interferon IFN-α in the peripheral blood of mice was measured by ELISA.

From Figure 9, it can be seen that amlexanox has no significant effect on the innate immune response caused by HBV virus infection, especially the induction of type I interferon.

**Implementing Example 10** Effect of amlexanox on hepatitis B virus in HepAD38 cell model.

$2x10^5$ HepAD38 cells that were stably expressing HBV with good growth status were seeded in collagen pretreated 6-well plates, and the corresponding concentrations of the test substances were added: final concentration of 1μM for entecavir (ETV) and a final concentration of 20μM, 10μM, and 5μM for amlexanox (AML), respectively. Two groups of controls were also set up, one with solvent only (DMF) and one with antibiotic only (tetracycline). Three replicate wells were set for all groups. After 96 hours and 144 hours of test article addition, 500 μL of cell culture supernatant was used to extract DNA from the supernatant, and the amount of HBV DNA in the supernatant was detected by qPCR. After 144h, cells were harvested and lysed in Lysis buffer A (50 mM Tris-HCl, pH 7.4, 1 mM EDTA, and 1% NP-40). The supernatant and pellet of the lysates were collected, respectively, from which DNA was extracted and quantified by quantitative PCR. Linear DNA was removed by DNA exonuclease (NEB) after DNA extraction from the cell lysate pellet, so only circular DNA (cccDNA) was examined (Figure 10). Viral DNA in the supernatant of cell lysates was defined as core particle DNA (Figure 11). Quantification of viral DNA in the cell culture supernatant is shown in Figure 12.

Results as shown in Figures 10, 11, and 12, it can be seen that amlexanox significantly inhibits hepatitis B virus replication in the HepAD38 cell model.

**Implementing Example 11** Effect of amlexanox on HBsAg and core antigen in cell models.

$2x10^5$ stably HBV-expressing HepAD38 cells with good growth status were seeded in collagen-pretreated 6-well plates, and the corresponding concentrations of the test substances were added: a final concentration of 1μM of entecavir (ETV) and a final concentration of 2 μM, 10μM, and 5μM of amlexanox (AML), respectively. Two groups of controls were also set up, one with only solvents (DMF) and one with antibiotics (tetracycline). Three replicate wells were set up for all experiments. 500μL of culture supernatant was taken at 24h, 48h, 96h, and 144h after adding the test article for ELISA to

detect HBsAg concentration (Figure 13).

2x$10^5$ stably HBV-expressing HepAD38 cells with good growth status were seeded in collagen-pretreated 6-well plates and added with the corresponding concentrations of the test substances: entecavir (ETV) at a final concentration of 1μM and amlexanox (AML) at final concentrations of 16μM, 10μM, 8μM, 5μM, 4μM, 2.5μM, and 2μM, respectively. A solvent-only control group (DMF) was also set up. Three replicate wells were set up for all experiments. 500μL of culture supernatant was taken at 24h, 48h, 72h, 96h, and 144h after adding the test article for ELISA to detect HBV core antigen (HBeAg) concentration. (Figure 14)

As shown in Figures 13 and 14, amlexanox could significantly reduce HBsAg level and core antigen level in the HepAD38 cell model.

**Implementing Example 12** Effect of amlexanox in a mouse model of venous hypertension.

Six-week-old male C57 mice were rapidly injected with 10μg/mouse of viral genomic DNA (genotype A) by tail vein injection in a short period. Viral infection was established using a venous hypertension model and randomly divided into groups. From the second day after injection, according to the concentration-time curve of the compound, oral gavage administration of entecavir (ETV, 0.03 mg/kg/day, once a day), or the specified concentration of amlexanox (equivalent to the adult dose of 25, 50, 100, 150, 200 mg/day, once a day) or an equal volume of solvent (model) at the same concentration. At the same time, a blank control was set up, and oral administration in uninfected mice was equivalent to the model group. The groups and doses administered to animals in each group are shown in the table below:

| Group | Drug | Dose | Number of animals |
|---|---|---|---|
| 1 Blank group | Solvent | | 18 |
| 2 Model group | Solvent | | 18 |
| 3 ETV | Entecavir | 0.03 mg/kg/day | 10 |
| 4 Test article dose group 1 | Amlexanox | 25 mg/day * | 10 |
| 5 Test article dose group 2 | Amlexanox | 50 mg/day * | 10 |
| 6 Test article dose group 3 | Amlexanox | 100 mg/day * | 10 |
| 7 Test article dose group 4 | Amlexanox | 150 mg/day * | 10 |
| 8 Test article dose group 5 | Amlexanox | 200 mg/day * | 10 |

*It indicates that the dose herein is the value converted into an adult dose. The conversion method is as follows: Assume adult weight 60 kg, adult dose/day = mouse dose/kg/day * 60 kg/9.1, for example, adult 25 mg/day is equivalent to mouse 3.8 mg/kg/day.

On days **1,** 3, and 7 of administration, an appropriate amount of blood was collected from the tail vein of mice in each group. On day 9, the remaining peripheral blood was collected after the mice were sacrificed. All blood samples were centrifuged to obtain serum, divided into two portions. One was used to detect the concentration of HBsAg by ELISA, and the other was used to detect the enzyme activity of alanine aminotransferase (ALT) by alanine aminotransferase kit (colorimetric method) (Nanjing Jiancheng Bioengineering Institute).

Results are shown in Figures 15 and 16. It can be seen from Figure 15 that seven days after continuous administration of amlexanox, the dose higher than 50 mg/day significantly reduced surface antigen (HBsAg) levels and inhibited HBV activity in mice. From Figure 16, it can be seen that amlexanox can effectively reduce alanine aminotransferase activity, indicating that amlexanox can effectively reduce the level of liver injury and has a repairing effect on venous hypertension and HBV-induced liver injury.

## Implementing Example 13

Six-week-old male C57 mice were rapidly injected with 10μg/mouse of viral genomic DNA (genotype A) by tail vein injection in a short period. Viral infection was established using a venous hypertension model and randomly divided into groups. From the second day after injection, according to the compound concentration-time curve, oral gavage administration of entecavir (ETV, 0.03 mg/kg/day, once a day), lamivudine (3TC, 530 mg/kg/day, once a day), tenofovir (TDF, 530 mg/kg/day, once a day) or amlexanox (equivalent to the adult dose of 150 mg/day, once a day) or an equal volume of solvent(model) was performed. At the same time, a blank control was set up, and oral administration in uninfected mice was equivalent to the model group. The groups and doses administered to animals in each group are shown in the table below:

| | Group | Drug | Dose | Number of animals |
|---|---|---|---|---|
| 1 | Blank group (NC) | Solvent | | 18 |
| 2 | Model group | Solvent | | 18 |
| 3 | 3TC | Lamivudine | 530 mg/kg/day | 10 |
| 4 | ETV | Entecavir | 0.03 mg/kg/day | 10 |
| 5 | TDF | Tenofovir | 530 mg/kg/day | 10 |
| 6 | Test article low dose group (A150) | Amlexanox | 150 mg/day * | 10 |
| *It indicates that the dose herein is the value converted into an adult dose. The conversion method is as follows: assuming an adult weight of 60kg, the adult dose/day = mouse dose/kg/day * 60 kg/9.1. 150 mg/day in adults is equivalent to 22.8 mg/kg/day in mice. | | | | |

On days 5 and 7 of administration, an appropriate amount of blood was collected from the tail vein of mice in each group. On day 9, the remaining peripheral blood was collected after the mice were sacrificed. All blood samples were centrifuged to obtain serum, and the concentration of HBsAg was measured by ELISA. The specific method was based on the instructions of the ELISA kit.

The results are shown in Figure 17 that the HBsAg level in the blood was significantly decreased after continuous administration of 150 mg/day of amlexanox for five days, indicating that 150 mg/day of amlexanox had good anti-hepatitis B activity.

**Implementing Example 14** Effect of amlexanox in a transgenic mouse model.

Ten male C57BL/6-HBV transgenic mice (provided by Beijing Vitalstar Biotechnology Co., Ltd.) aged six weeks were randomly divided into two groups (control group, n=5; administration group, n=5). The doses administered to animals in each group are shown in the table below:

| Group | Drug | Dose (mg/kg) | Number of animals |
|---|---|---|---|
| 1 Control (NC) | 10% β-cyclodextrin solution | | 5 |
| 2 Experimental group (Aml) | Amlexanox | 48 | 5 |

Route of administration: Intragastric administration, 1mL sterile syringe was used for administration.

Dosing frequency: The drug was administered once daily for 35 consecutive days, with free access to food and water.

Dose-volume: 10 mL/kg. The dose-volume for each animal was determined based on the most recent body weight before administration.

On the 7th, 14th, 21st, and 35th day of administration, an appropriate amount of blood was collected from the tail vein of mice in each group. The serum was separated and diluted 5000 times with PBS, respectively. The ELISA kit detected the concentration of HBV surface antigen (HBsAg). The OD value at 450 nm was measured on a microplate reader.

The inhibition rate was calculated according to the following formula:

$$\text{Inhibition rate} = [(\text{OD450 control well} - \text{OD450 dose well})/\text{OD450 control well}] \times 100\%$$

The efficacy of amlexanox was evaluated in HBV transgenic mouse models. The HBV surface antigen HBsAg detection results are shown in Figure 18 that amlexanox could inhibit HBsAg level in transgenic mice at a dose concentration of 48 mg/kg.

**Implementing Example 15** Effect of amlexanox on immune cells in mice.

Six-week-old male C57 mice were rapidly injected with 10μg/mouse of viral genomic DNA (genotype A) by tail vein injection in a short period. Viral infection was established using a venous hypertension model and randomly divided into groups. From the second day after injection, according to the compound concentration-time curve, oral gavage administration of lamivudine (3TC, 530 mg/kg/day, once a day), tenofovir (TDF, 530 mg/kg/day, once a day), entecavir (ETV, 0.03 mg/kg/day, once a day), or designated concentrations of amlexanox (4, 8, 16 mg/kg/time, twice a day) or an equal volume of an equal concentration of solvent (model) was performed. At the same time, a blank control was set up, and oral administration in uninfected mice was equivalent to the model group. The administration dose in the groups and groups is shown in the table below:

| Group | Drug | Dose (mg/kg) | Number of animals |
|---|---|---|---|
| 1 Control group (con) | Solvent | | 18 |
| 2 Model group | Solvent | | 18 |
| 3 3TC | Lamivudine | 530 | 10 |
| 4 TDF | Tenofovir | 530 | 10 |
| 5 ETV | Entecavir | 0.03 | 10 |
| 4. Test article low dose group (4 mg) | Amlexanox | 4 (twice/day) | 10 |
| 5 Test article median dose group (8 mg) | Amlexanox | 8 (twice/day) | 10 |
| 6 Test article high dose group (16 mg) | Amlexanox | 16 (twice/day) | 10 |

On day 9, mice were sacrificed to collect blood stained with flow antibody after fission red. All cells were treated with anti-FcγRIII/ II (purchased from BD Biosciences) in PBS for 30 min. CD3e-FITC (purchased from BD Biosciences). CD4-PE, CD8a-APC-Vio770, and CD34-FITC (GE). CD19-APC, CD11b-PE-Cyanine7, and CD117-efflor 450 (purchased from eBioscience). After staining, the samples were suspended using 500μL PBS and analyzed by BD Aria III flow cytometer.

The results in Figure 19 show that amlexanox had an inhibitory effect on both B and T cells, and the inhibitory effect was dose-dependent. Additionally, lamivudine also had some inhibitory effects on immune cells. With the increase of concentration, the inhibition of immune cells increased. Still, the inhibitory effect on HBV instead increased, indicating that amlexanox did not exert an anti-HBV effect through the immune system.

**Implementing Example 16** Cytotoxicity test.

The cytotoxicity of amlexanox in HepG2, HCT-116, Hela, A549, MCF7, MKN45, THP-1, Huh7, and 293 cells was tested by MTT assay. Various cells in the logarithmic growth phase were seeded in 96-well plates at 6,000 cells/well and cultured for 24h. Different concentrations of amlexanox were added to the plates (final concentrations of 100, 50, 25, 12.5, 6.25, and 3.125μM ), positive control doxorubicin hydrochloride (final concentration of 1μM ), the blank control group was added with an equal volume of culture medium. The dosage of DMSO in the solvent control group was based on the maximum dose of 0.1% used in the test group, with four duplicate wells for each concentration. Cells were treated with the drug for 72h and then added with 20μL 5 mg/mL MTT, cultured at 37°C for 4h, after added 100μL triple solution (10% SDS, 5% isobutanol, 12 mM hydrochloric acid), 96-well cell culture plate was placed in an incubator containing 5% CO2 at 37 ± 1°C overnight. OD value at 570nm was measured on a microplate reader.

The inhibition rate of tumor cell growth was calculated according to the following formula:

Inhibition rate (%) = [(OD570 control well - OD570 dose well)/OD570 control well] × 100.

The test results are shown in Table 1,

Table 1

| Grouping | Concentration (μM) | Inhibition (%) | | | | | | | | |
|---|---|---|---|---|---|---|---|---|---|---|
| | | Hep G2 | HC-T-1 16 | Hel a | A5 49 | MC F7 | MKN 45 | THP -1 | Hu h7 | 293 |
| **Adriamycin Hydrochloride** | 1 | 92.07 | 71.93 | 48. 8 | 85. 79 | 87.9 7 | 39.38 | 78.0 8 | 76.9 | 57. 94 |
| **AML** | 100 | 4.8 | 31.15 | 6.1 5 | 4.5 4 | 43.5 5 | 30.86 | 7.44 | 35.2 2 | 11. 26 |
| | **50** | 0 | 6.41 | 1.8 9 | 1.0 8 | 16.4 2 | 13.94 | 0 | 26.2 2 | 0 |
| | **25** | 0 | 1.17 | 0.7 9 | 0 | 3.8 | 9.22 | 0 | 18.0 5 | 0 |
| | **12.5** | 0 | 0.11 | 1.7 | 0.6 5 | 1.22 | 4.9 | 0 | 15.0 3 | 0 |
| | **6.25** | 0 | 0 | 2.4 8 | 1.3 1 | 0.86 | 3.61 | 0 | 7.28 | 0 |
| | **3.125** | 0 | 0 | 3.0 8 | 3.5 5 | 1.03 | 0 | 0 | 0 | 0 |

It can be seen that the compound of the invention has no obvious cytotoxicity in the nine kinds of cells tested below

the concentration of 50μM. At 100μM concentration, MKN45, HCT-116, MCF7, and Huh7 cells have some cytotoxicity, and no significant cytotoxicity is seen in the remaining five cells.

All literature mentioned in the invention is referred to in the application as if each article had been cited separately as a reference. It is also understood that having read the previous teaching contents of the invention, the technicians in this field may make various alterations or modifications to the invention, and these equivalent forms also fall within the scope of the claims annexed to the application.

## Claims

1. Amlexanox for use in the prevention and/or treatment of hepatitis virus infection or in the prevention and/or treatment of diseases caused by hepatitis virus infection, wherein the disease caused by that hepatitis virus infection is chosen from acute hepatitis, chronic hepatitis, liver cirrhosis, liver fibrosis, or liver cancer.

2. Amlexanox for use according to claim **1,** wherein said hepatitis virus is hepatitis B virus.

3. Amlexanox for use according to claim **1,** wherein the disease caused by the hepatitis virus infection results in liver damage.

4. A pharmaceutical composition **characterized by**:

    1) Therapeutic effective doses of amlexanox, and
    2) one or more active antiviral substances at therapeutically effective doses, wherein said active antiviral substance is a nucleoside or a nucleotide analog, and
    3) Pharmaceutically acceptable carrier,

    wherein said one or more nucleosides or nucleotide analogs are lamivudine, zidovudine, stavudine, tenofovir, entecavir, abacavir, zalcitabine, emtricitabine, dideoxyinosine, nevirapine, delavirdine, efavirenz, etravirine, and rilpivirine.

5. The pharmaceutical composition according to claim 4, **characterized in that** the nucleoside or nucleotide analog is one or more of lamivudine, tenofovir, or entecavir.

6. The pharmaceutical composition as defined in claims 4-5 for use in the prevention and/or treatment of hepatitis virus infection, or in the prevention and/or treatment of diseases caused by hepatitis virus infection, wherein the disease caused by that hepatitis virus infection is chosen from acute hepatitis, chronic hepatitis, liver cirrhosis, liver fibrosis, or liver cancer.

7. The pharmaceutical composition as defined in claims 4-5, for use according to claim 6, wherein the hepatitis virus is hepatitis B virus.

## Patentansprüche

1. Amlexanox zur Verwendung bei der Vorbeugung und/oder Behandlung einer Hepatitis-Virusinfektion oder bei der Vorbeugung und/oder Behandlung von Krankheiten, die durch eine Hepatitis-Virusinfektion verursacht werden, wobei die durch diese Hepatitis-Virusinfektion verursachte Krankheit ausgewählt ist aus akuter Hepatitis, chronischer Hepatitis, Leberzirrhose, Leberfibrose oder Leberkrebs.

2. Amlexanox zur Verwendung nach Anspruch 1, wobei das Hepatitis-Virus das Hepatitis-B-Virus ist.

3. Amlexanox zur Verwendung nach Anspruch 1, wobei die durch die Hepatitis-Virusinfektion verursachte Krankheit zu einer Leberschädigung führt.

4. Pharmazeutische Zusammensetzung, **gekennzeichnet durch**:

    1) Therapeutisch wirksame Dosen von Amlexanox, und
    2) eine oder mehrere aktive antivirale Substanzen in therapeutisch wirksamen Dosen, wobei die aktive antivirale

Substanz ein Nukleosid oder ein Nukleotidanalogon ist, und
3) Pharmazeutisch akzeptabler Träger,

wobei das eine oder die mehreren Nukleoside oder Nukleotidanaloga Lamivudin, Zidovudin, Stavudin, Tenofovir, Entecavir, Abacavir, Zalcitabin, Emtricitabin, Dideoxyinosin, Nevirapin, Delavirdin, Efavirenz, Etravirin und Rilpivirin sind.

5. Pharmazeutische Zusammensetzung nach Anspruch 4, **dadurch gekennzeichnet, dass** das Nukleosid oder Nukleotidanalogon eines oder mehrere von Lamivudin, Tenofovir oder Entecavir ist.

6. Pharmazeutische Zusammensetzung, wie in den Ansprüchen 4-5 definiert, zur Verwendung bei der Vorbeugung und/oder Behandlung einer Hepatitis-Virusinfektion oder bei der Vorbeugung und/oder Behandlung von Krankheiten, die durch eine Hepatitis-Virusinfektion verursacht werden, wobei die Krankheit, die durch diese Hepatitis-Virusinfektion verursacht wird, ausgewählt ist aus akuter Hepatitis, chronischer Hepatitis, Leberzirrhose, Leberfibrose oder Leberkrebs.

7. Pharmazeutische Zusammensetzung gemäß den Ansprüchen 4-5 zur Verwendung nach Anspruch 6, wobei das Hepatitis-Virus das Hepatitis-B-Virus ist.

## Revendications

1. Amlexanox pour la prévention et/ou le traitement de l'infection par le virus de l'hépatite ou pour la prévention et/ou le traitement des maladies causées par l'infection par le virus de l'hépatite, la maladie causée par l' infection par le virus de l'hépatite étant choisie parmi l'hépatite aiguë, l'hépatite chronique, la cirrhose du foie, la fibrose du foie ou le cancer du foie.

2. Amlexanox pour utilisation selon la revendication 1, dans lequel ledit virus de l'hépatite est le virus de l'hépatite B.

3. Amlexanox pour utilisation selon la revendication 1, dans lequel la maladie causée par l'infection par le virus de l'hépatite entraîne des lésions hépatiques.

4. Composition pharmaceutique **caractérisée par**:

1) doses thérapeutiques efficaces d'amlexanox, et
2) une ou plusieurs substances antivirales actives à des doses thérapeutiques efficaces, ladite substance antivirale active étant un nucléoside ou un analogue de nucléotide, et
3) support pharmaceutiquement acceptable,

dans lequel un ou plusieurs nucléosides ou analogues de nucléotides sont la lamivudine, la zidovudine, la stavudine, le ténofovir, l'entécavir, l'abacavir, la zalcitabine, l'emtricitabine, la didésoxyinosine, la névirapine, la delavirdine, l'efavirenz, l'étravirine et la rilpivirine.

5. Composition pharmaceutique selon la revendication 4, **caractérisée par le fait que** le nucléoside ou l'analogue de nucléotide est un ou plusieurs des éléments suivants lamivudine, ténofovir ou entécavir.

6. Composition pharmaceutique telle que définie dans les revendications 4-5 pour utilisation dans la prévention et/ou le traitement de l'infection par le virus de l'hépatite, ou dans la prévention et/ou le traitement des maladies causées par l'infection par le virus de l'hépatite, dans laquelle la maladie causée par l'infection par le virus de l'hépatite est choisie parmi l'hépatite aiguë, l'hépatite chronique, la cirrhose du foie, la fibrose du foie, ou le cancer du foie.

7. Composition pharmaceutique telle que définie dans les revendications 4-5, pour l'utilisation selon la revendication 6, dans laquelle le virus de l'hépatite est le virus de l'hépatite B.

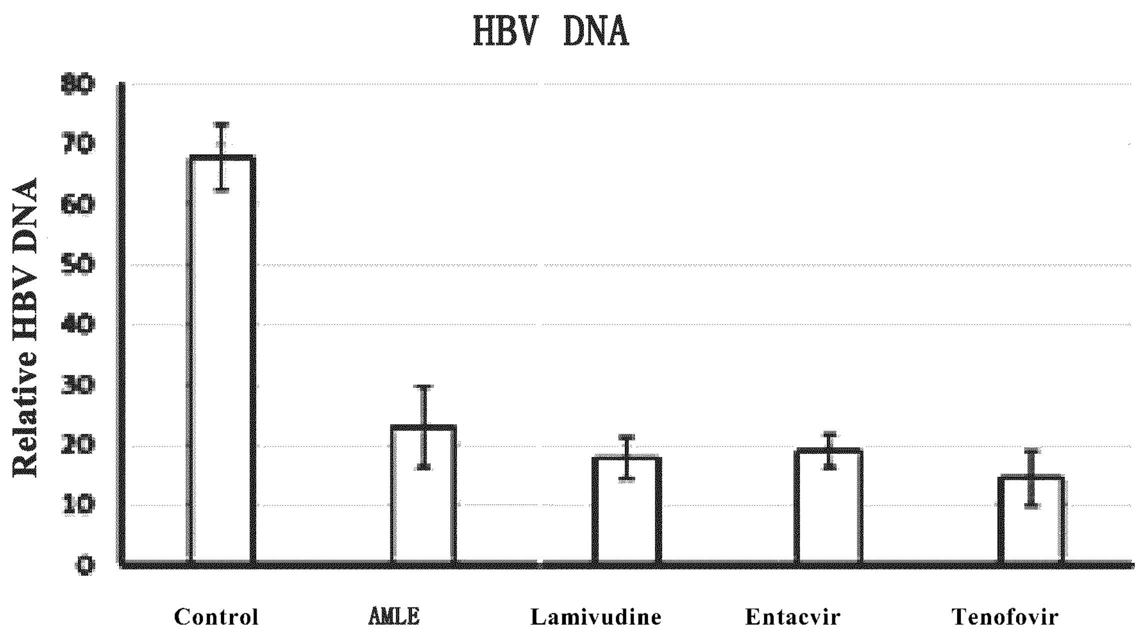


Figure 1

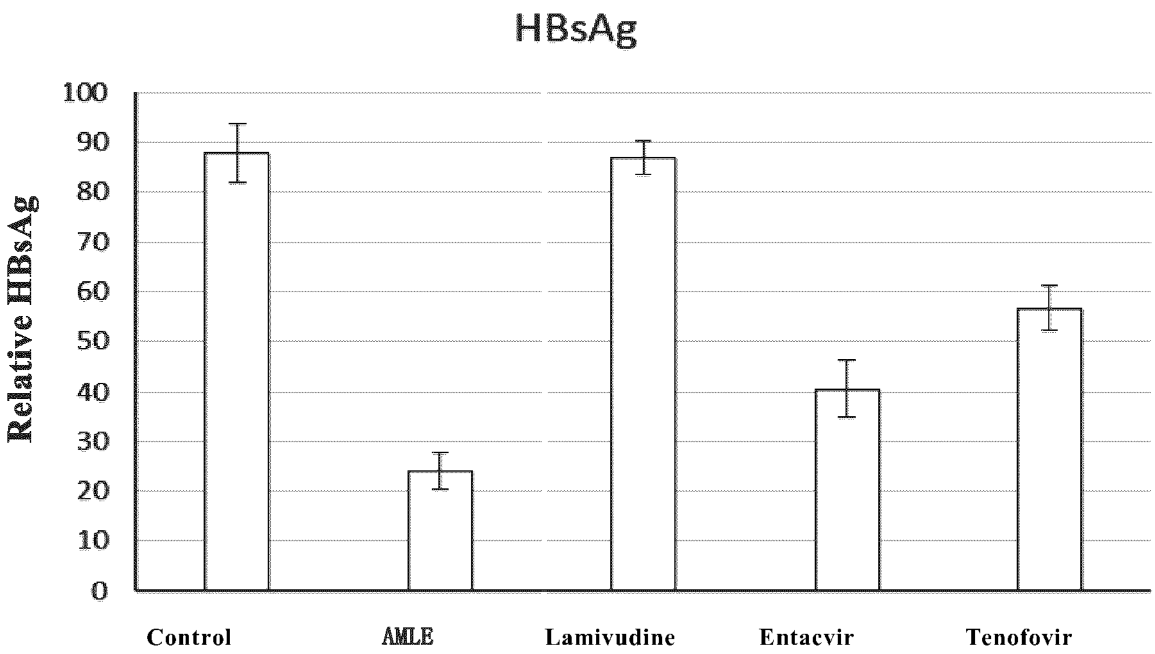


Figure 2

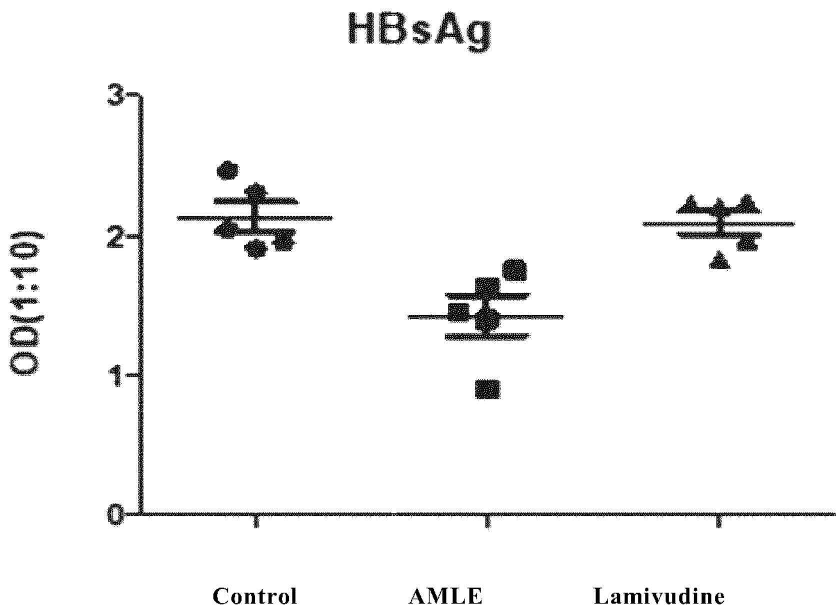


Figure 3

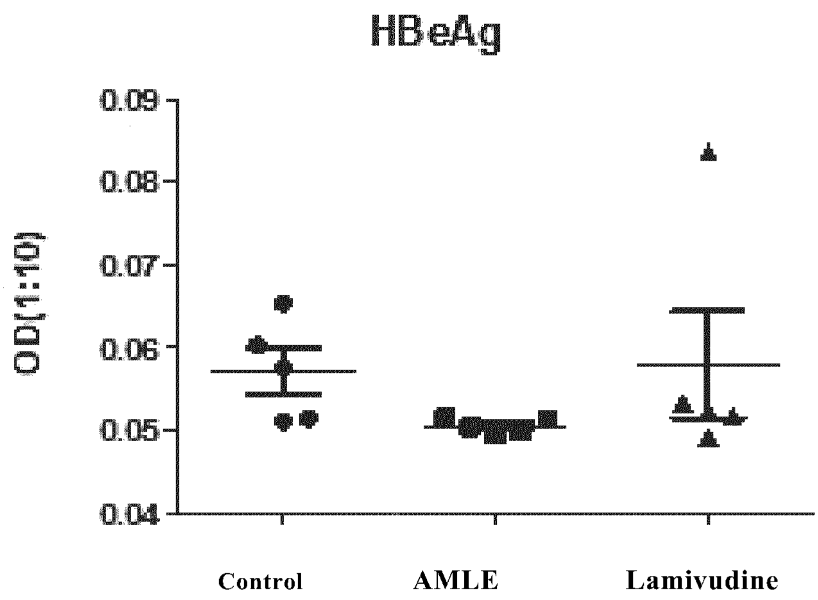


Figure 4

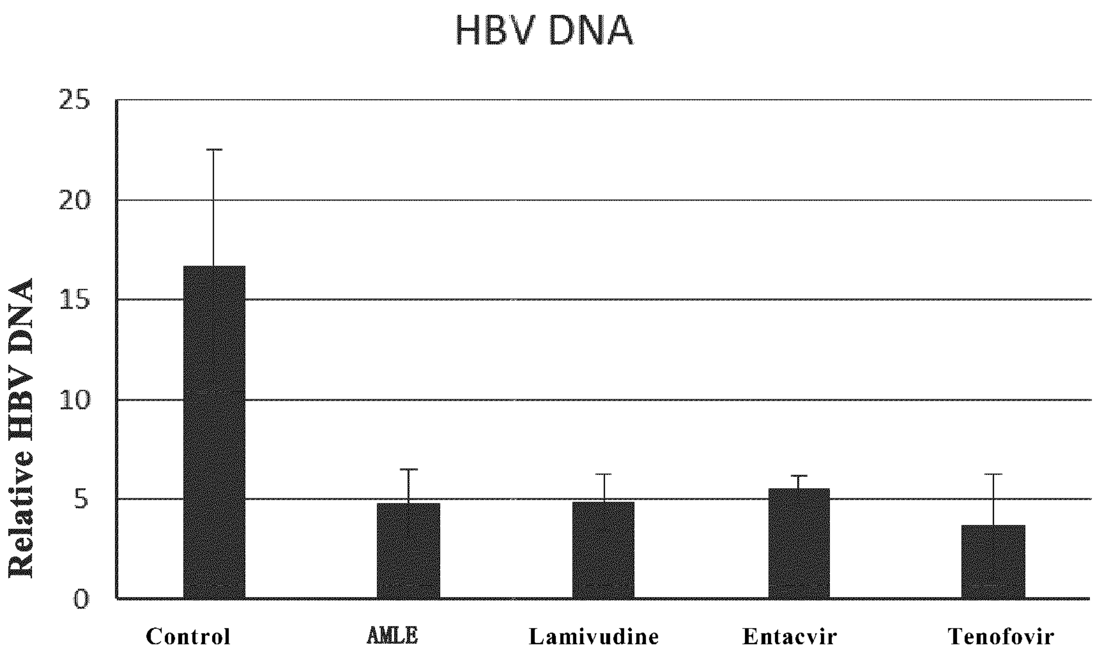


Figure 5

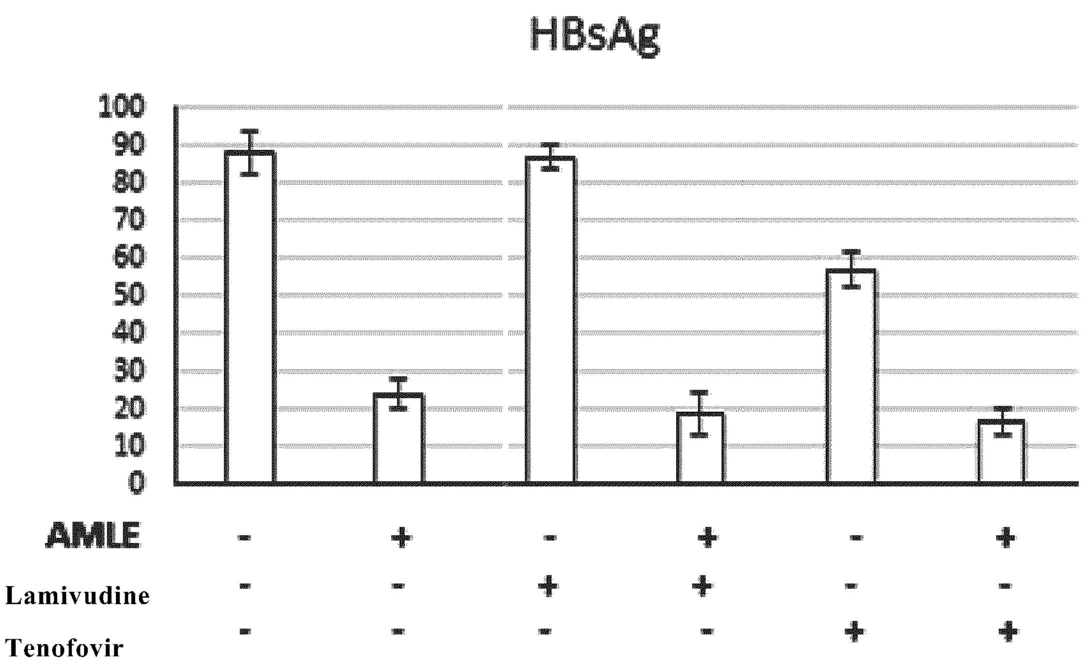


Figure 6

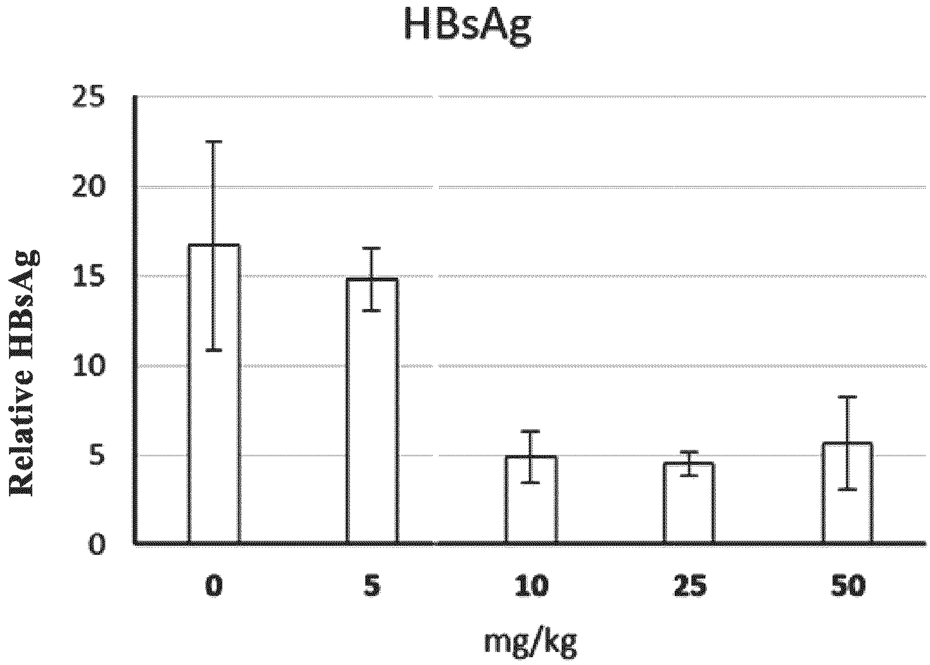


Figure 7

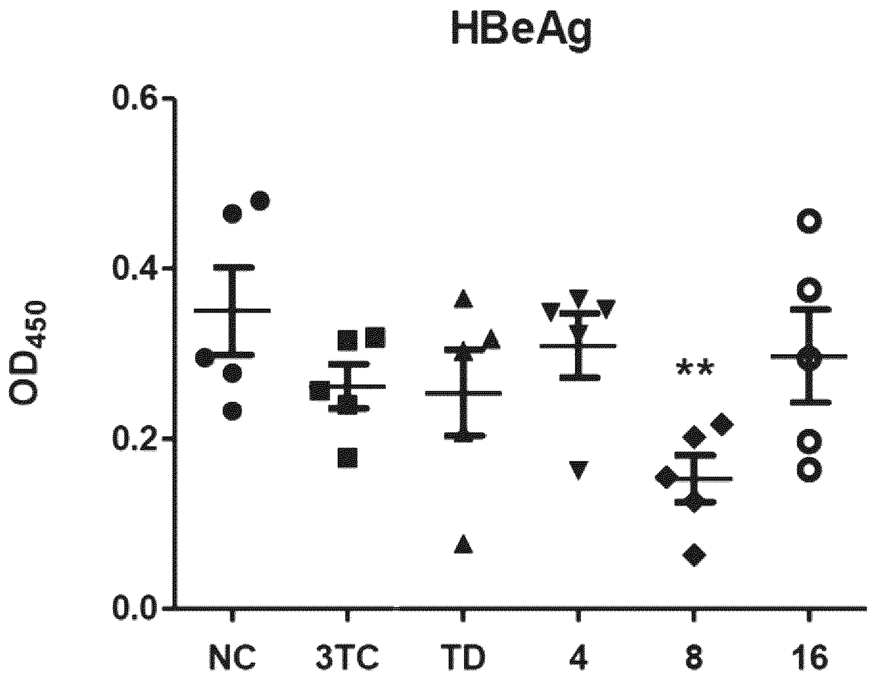


Figure 8

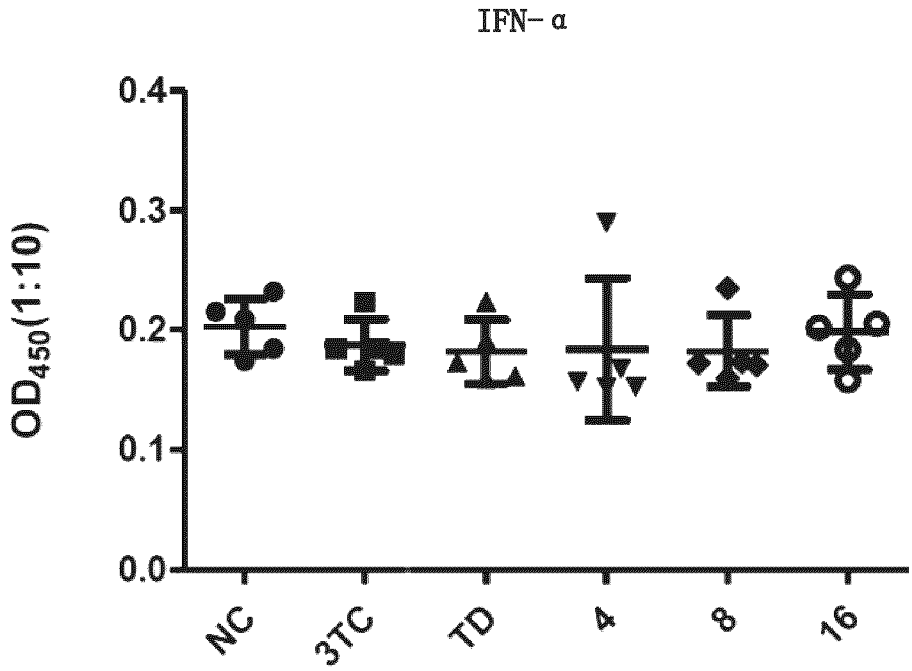


Figure 9

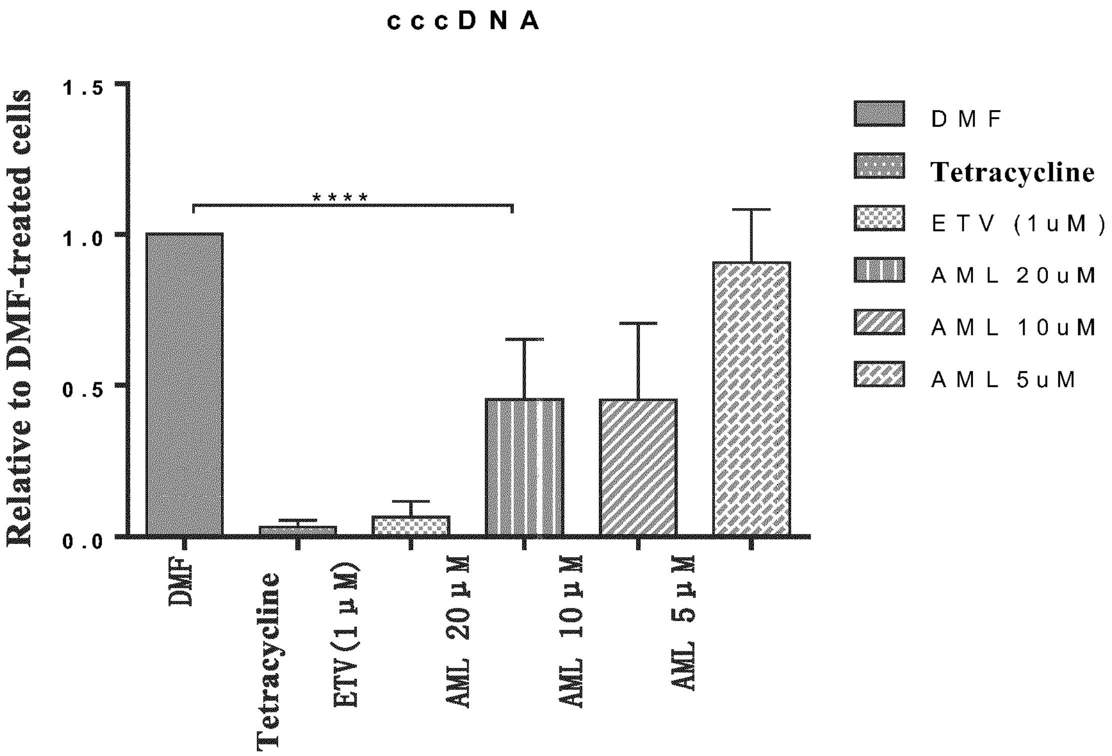


Figure 10

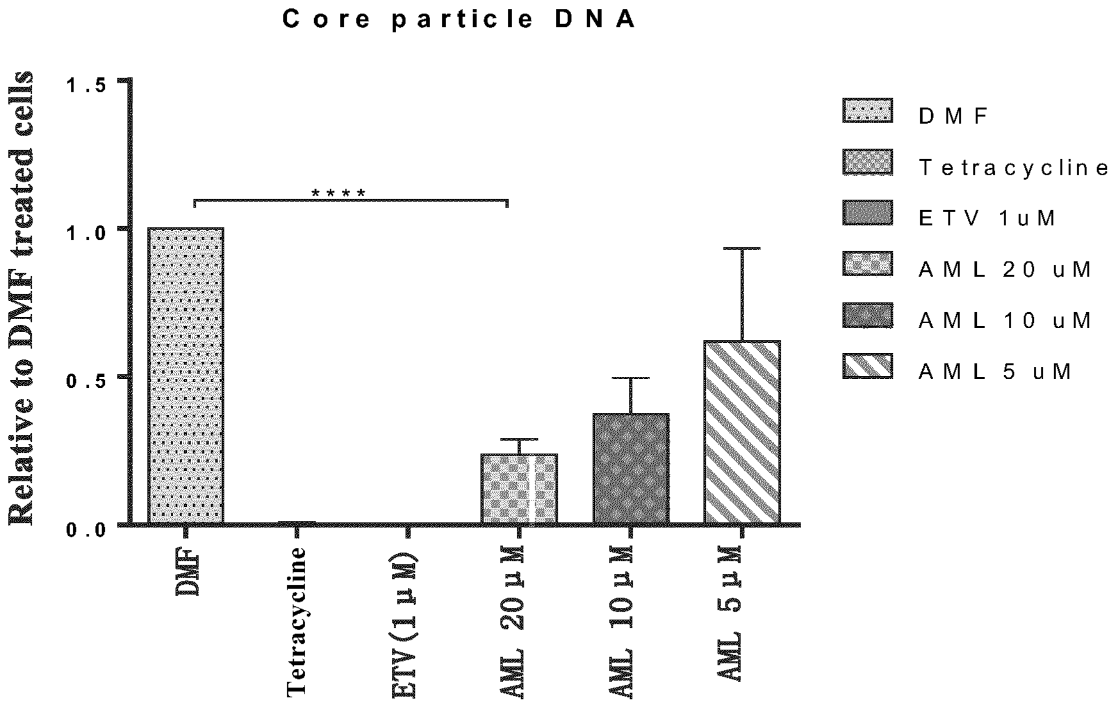


Figure 11

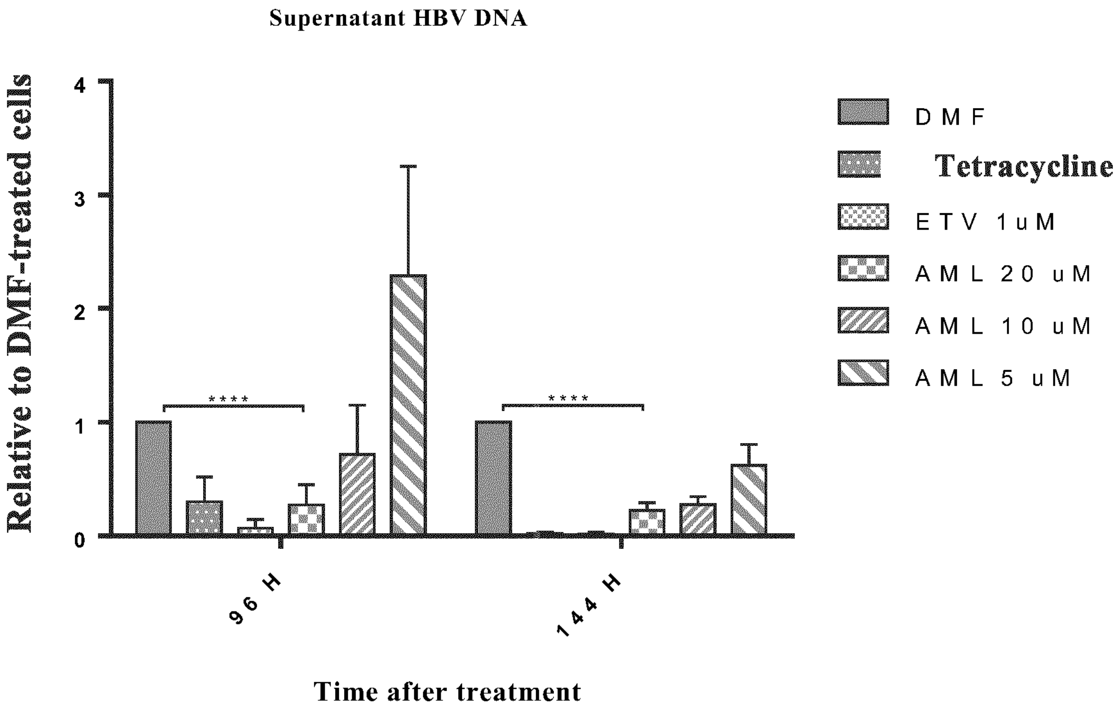


Figure 12

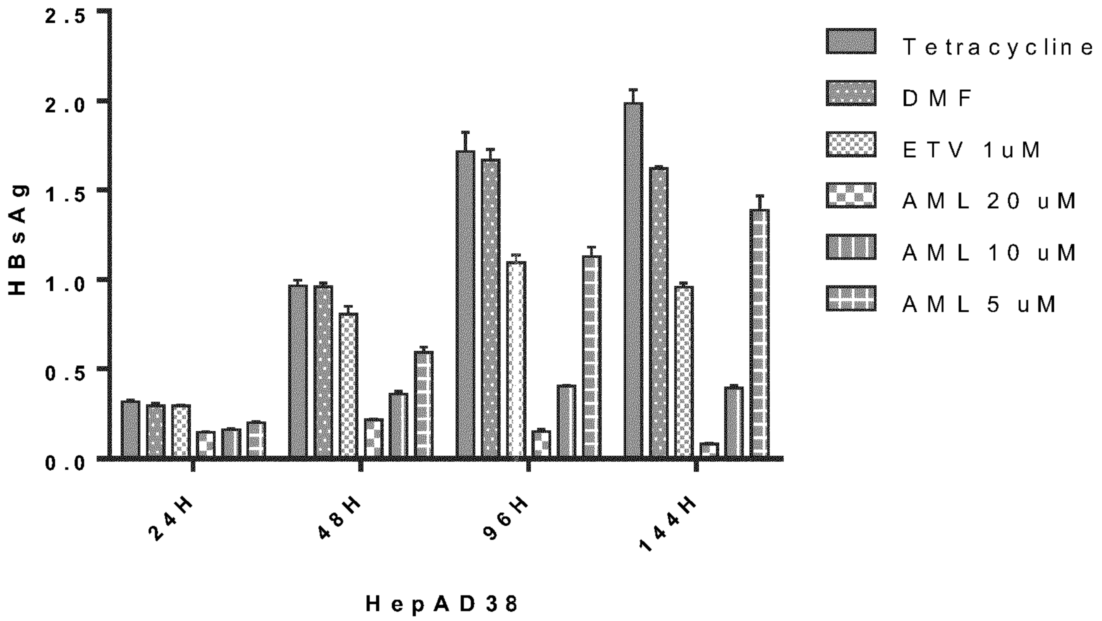


Figure 13

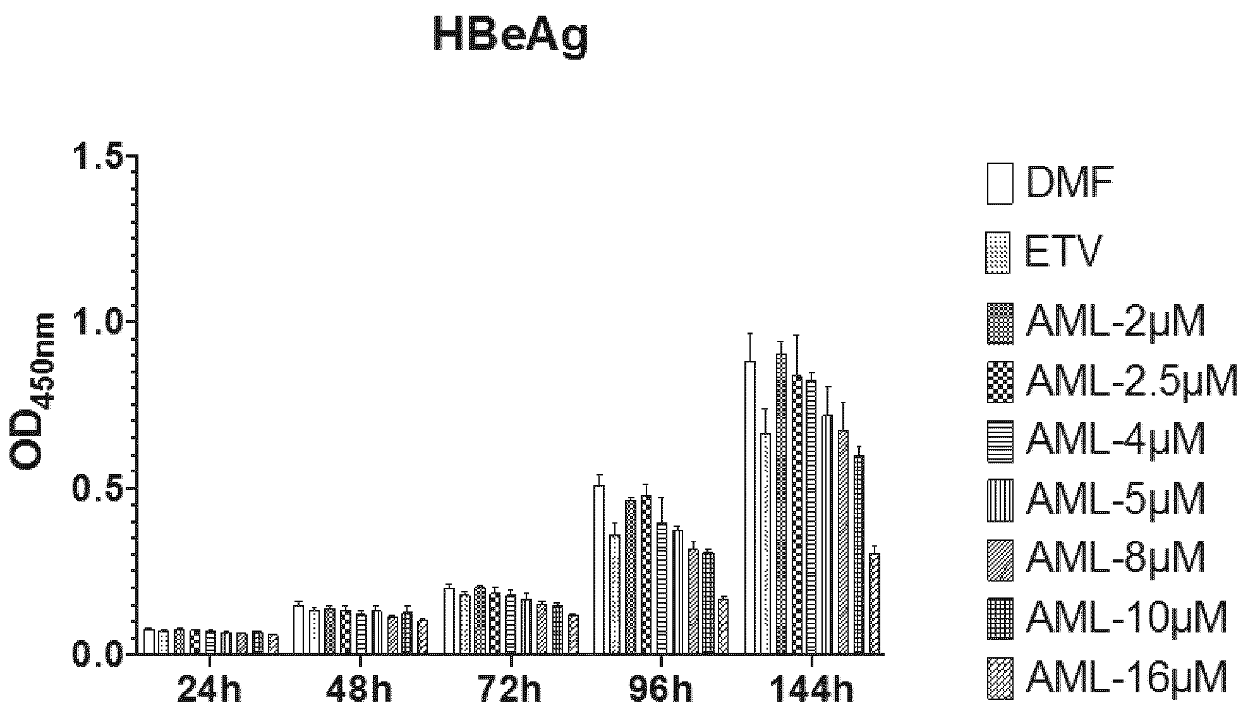


Figure 14

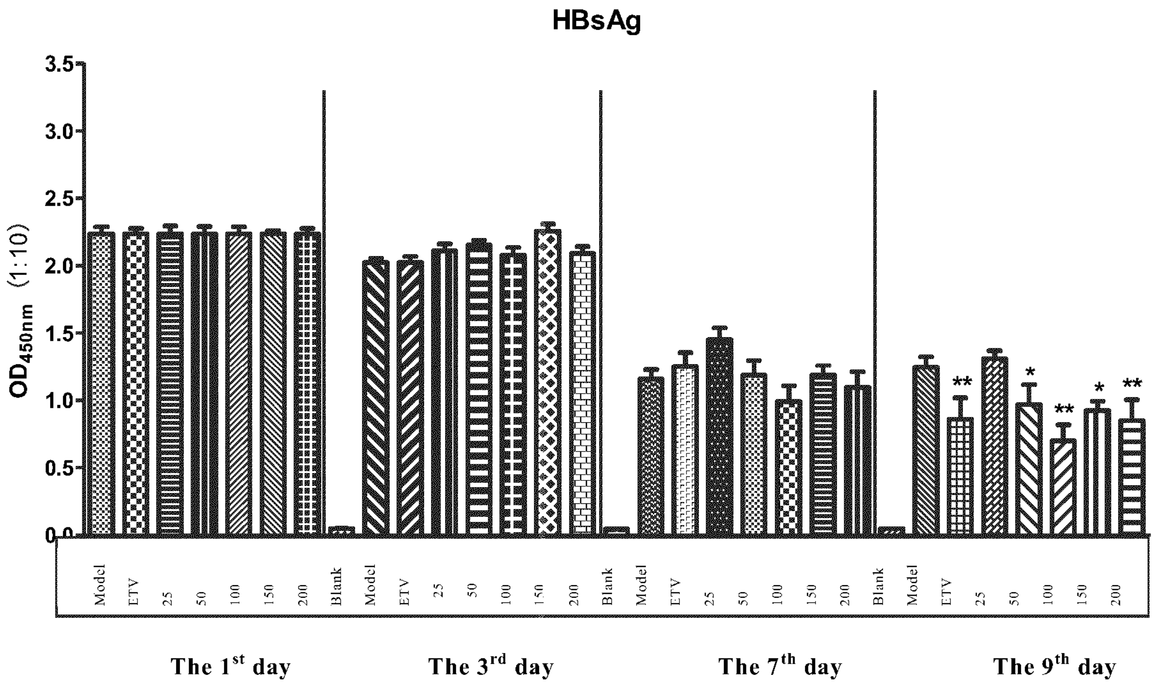


Figure 15

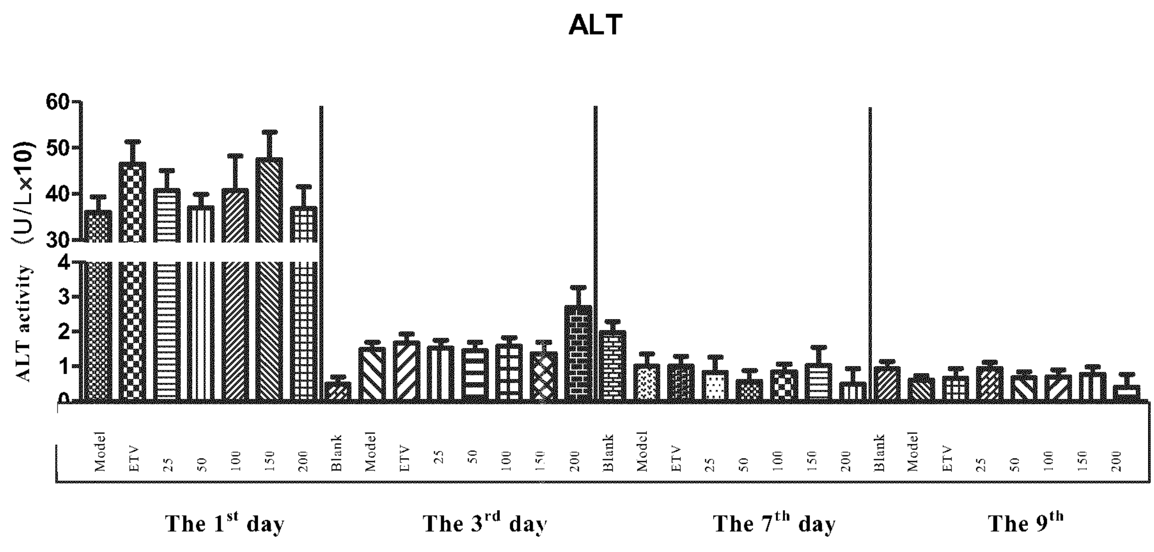


Figure 16

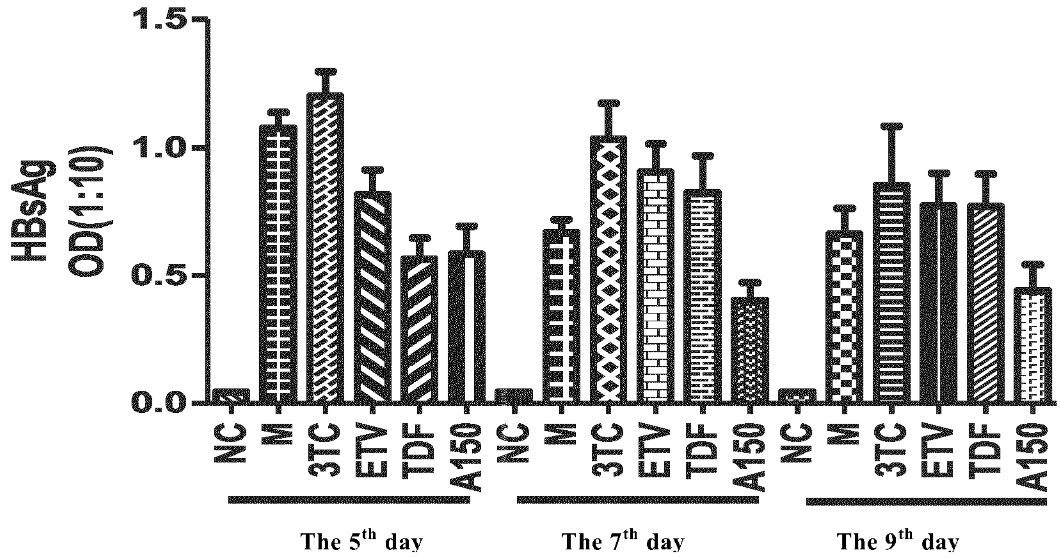


Figure 17

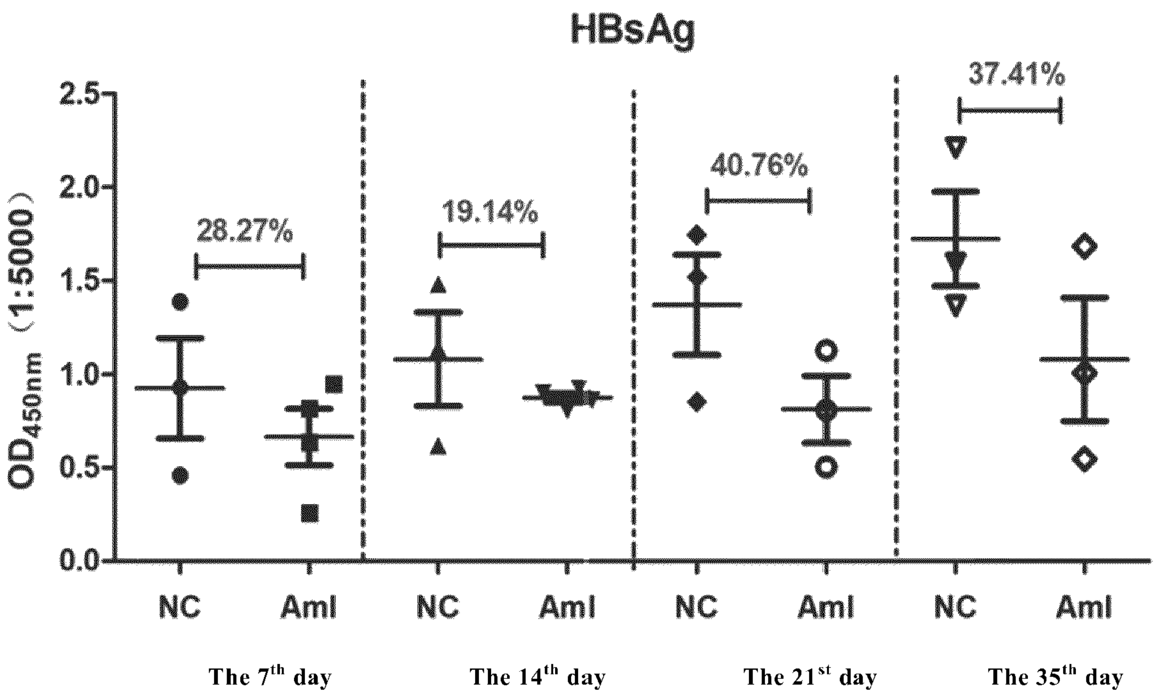


Figure 18

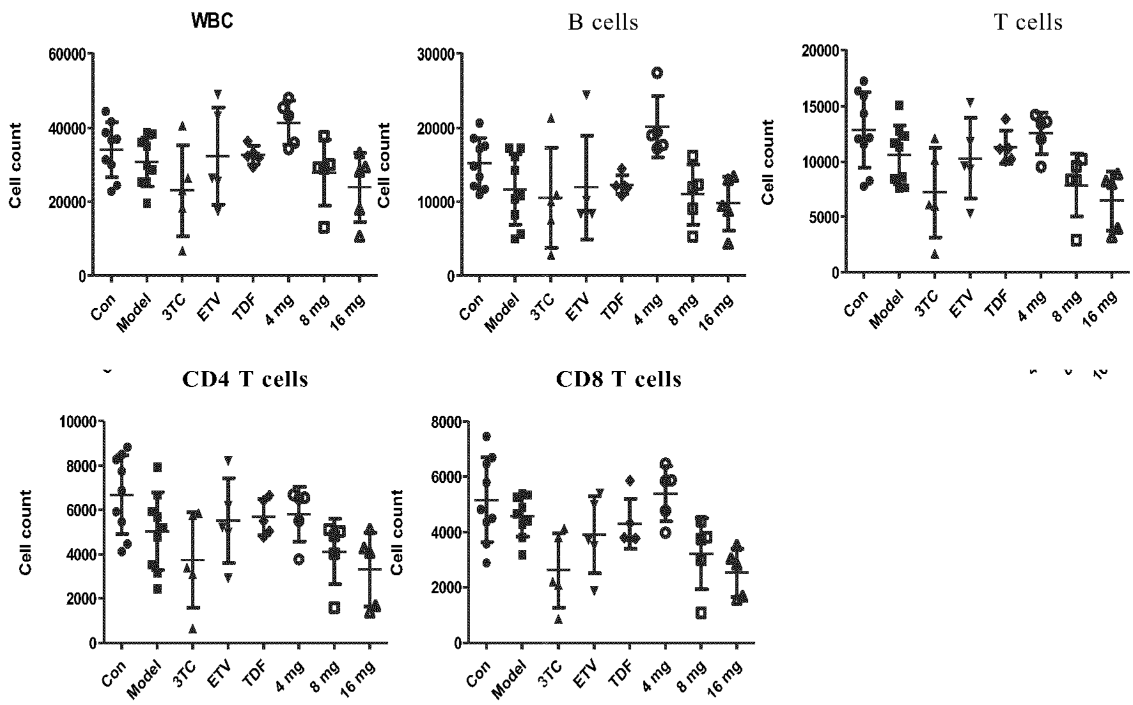

WBC
B cells
T cells
CD4 T cells
CD8 T cells
Cell count
Con
Model
3TC
ETV
TDF
4 mg
8 mg
16 mg


Figure 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016289684 A
- WO 2006133099 A
- WO 2016201243 A